(19) Europäisches Patentamt
European Patent Office
Office européen des brevets

(11) Veröffentlichungsnummer: **0 058 639**
**B1**

(12) # EUROPÄISCHE PATENTSCHRIFT

(45) Veröffentlichungstag der Patentschrift: **14.08.85**

(51) Int. Cl.⁴: **C 07 D 241/44**, A 01 N 43/60

(21) Anmeldenummer: **82810058.6**

(22) Anmeldetag: **10.02.82**

(54) **2-(4-(6-Halogen-chinoxalinyl-2-oxy)-phenoxy)-propionsäureester, Verfahren zu ihrer Herstellung sowie ihre Verwendung als Herbizide und Pflanzenwuchsregulatoren.**

(30) Priorität: **16.02.81 CH 1009/81**

(43) Veröffentlichungstag der Anmeldung: **25.08.82 Patentblatt 82/34**

(45) Bekanntmachung des Hinweises auf die Patenterteilung: **14.08.85 Patentblatt 85/33**

(84) Benannte Vertragsstaaten: **AT BE CH DE FR GB IT LI NL**

(56) Entgegenhaltungen:
**EP - A - 0 023 785**
**EP - A - 0 026 622**
**EP - A - 0 046 467**
**CH - A - 622 170**
**DE - A - 3 004 770**

(73) Patentinhaber: **CIBA-GEIGY AG, Postfach, CH-4002 Basel (CH)**

(72) Erfinder: **Rempfler, Hermann, Dr., Brücklismattstrasse 16, CH-4107 Ettingen (CH)**
Erfinder: **Böhner, Beat, Dr., Hügelweg 3, CH-4102 Binningen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 058 639 B1

ACTORUM AG

**Beschreibung**

Die vorliegende Erfindung betrifft neue herbizid und pflanzenwuchsregulierend wirksame Ester von 2-[4- (6-Halogen-chinoxalinyl -2- oxy) -phenoxy] -propionsäuren, Verfahren zu ihrer Herstellung, ferner Mittel, die diese Verbindungen als Wirkstoffe enthalten, sowie die Verwendung der neuen Wirkstoffe und der sie enthaltenden Mittel zur selektiven Bekämpfung von Unkräutern in Kulturpflanzenbeständen und zur Regulierung des Pflanzenwachstums.

In den letzten Jahren sind zahlreiche Derivate von para-substituierten Phenoxypropionsäuren mit herbiziden Eigenschaften bekannt geworden, vgl. z.B.: DOS 2 609 461, DOS 3 004 770, EPA 23 785 und CH-PS 622 170.

Solche Verbindungen sind als Herbizide vorgeschlagen und praktisch verwendet worden, um zur Erleichterung der Landarbeit beizutragen und die Produktivitäten von landwirtschaftlichen Nutzpflanzen und Gartenbaunutzpflanzen zu verbessern.

Es besteht aber immer noch ein Bedürfnis, neue Herbizide zu finden, welche überlegene Herbizideigenschaften aufweisen. Herbizide, die für landwirtschaftliche und gartenbauliche Zwecke eingesetzt werden sollen, sind vorzugsweise Verbindungen, die bei Anwendung kleiner Dosismengen die Zielunkräuter selektiv bekämpfen, ohne dabei gegenüber Nutzpflanzen toxisch zu sein. Bekannte Herbizide weisen diese optimalen Herbizideigenschaften nicht immer auf.

Es wurde nun überraschenderweise gefunden, dass die neuen Wirkstoffe gemäss vorliegender Erfindung den in den Handel eingeführten Produkten und den strukturell nächst verwandten Verbindungen der Patentliteratur in der selektiven Unkrautbekämpfung überlegen sind und darüberhinaus pflanzenwuchsregulierende Eigenschaften besitzen.

Die erfindungsgemässen 2-[4- (6-Halogen-chinoxalinyl -2- oxy) -phenoxy] -propionsäureester entsprechen der allgemeinen Formel I,

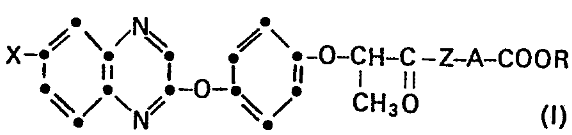

(I)

worin X Fluor, Chlor oder Brom, Z Sauerstoff oder Schwefel, A eine gegebenenfalls durch Methyl oder Äthyl substituierte $C_1$-$C_4$-Alkylenbrücke und R $C_1$-$C_4$-Alkyl bedeuten.

Alkyl steht für Methyl, Äthyl, n-Propyl, i-Propyl sowie die isomeren Butylreste.

Beispiele für Alkylenbrücken sind Methylen, Äthylen, Propylen und Butylen.

Wegen ihrer Wirkung bevorzugt sind Verbindungen der Formel I, in denen

a) X Fluor oder Chlor und

b) A eine gegebenenfalls durch Methyl oder Äthyl substituierte $C_1$-$C_2$-Alkylenbrücke bildet, die insgesamt höchstens 3 Kohlenstoffatome enthält.

Besonders bevorzugt sind die Verbindungen der Formel I, in denen X Fluor oder Chlor und A eine gegebenenfalls durch Methyl oder Äthyl substituierte $C_1$-$C_2$-Alkylenbrücke bildet, die insgesamt höchstens 3 Kohlenstoffatome enthält.

Als bevorzugte Einzelverbindungen sind zu nennen:

2- [4- (6-Chlor-chinoxalinyl -2- oxy) -phenoxy] -thiopropionsäure- methoxycarbonylmethylester,

2- [4- (6-Fluorchinoxalinyl -2- oxy) -phenoxy] -propionsäure- äthoxy- carbonylmethylester,

2- [4- (6-Fluorchinoxalinyl -2- oxy) -phenoxy] -thiopropionsäure- methoxy- carbonylmethylester,

2- [4- (6-Fluorchinoxalinyl -2- oxy) -phenoxy] -propionsäure -1- (methoxy- carbonyl)-äthylester,

2- [4, (6-Fluorchinoxalinyl -2- oxy) -phenoxy]- thiopropionsäure -n- butyloxy- carbonylmethylester,

2- [4- (6-Chlor-chinoxalinyl -2- oxy) -phenoxy] -propionsäure -1- (äthoxycarbonyl) -n-propylester und

2- [4- (6-Chlor-chinoxalinyl -2- oxy) -phenoxy] -propionsäure -1- (n-butyloxycarbonyl)-äthylester.

Zur Herstellung der neuen Ester der Formel I dienen an sich bekannte Verfahren:

Nach einem ersten erfindungsgemässen Verfahren werden die Verbindungen der Formel I erhalten, indem man ein Säurehalogenid der Formel II,

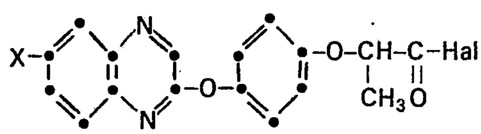

(II)

worin X die unter Formel I angegebene Bedeutung hat und Hal für Chlor oder Brom steht, gegebenenfalls in einem inerten Lösungsmittel und gegebenenfalls in Gegenwart einer Base mit einem Alkancarbonsäureester der Formel III

H–Z–A–COOR (III)

umsetzt, worin A, R und Z die unter Formel I gegebene Bedeutung haben.

Nach einem zweiten erfindungsgemässen Verfahren stellt man die Verbindungen der Formel I her, indem man ein Chinoxalinylchlorid der Formel IV,

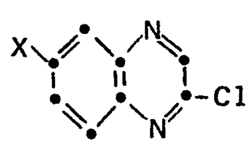

(IV)

worin X die unter Formel I angegebene Bedeutung hat, in einem inerten Lösungsmittel, gegebenenfalls in Gegenwart einer Base, mit einem Phenoxypropionsäureester der Formel V

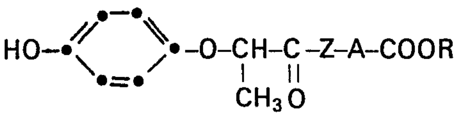 (V)

umsetzt, worin A, R und Z die unter Formel I angegebene Bedeutung haben.

Nach einem dritten erfindungsgemässen Verfahren kann man die Verbindungen der Formel I auch erhalten, indem man ein Chinoxalinyl -2-oxy-phenol der Formel VI,

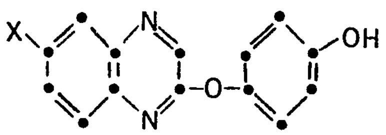 (VI)

worin X die unter Formel I angegebene Bedeutung hat, in einem inerten Lösungsmittel, gegebenenfalls in Gegenwart einer Base, mit einem Propionsäureester der Formel VII

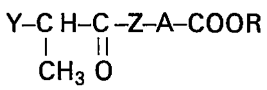 (VII)

umsetzt, worin A, R und Z die unter Formel I angegebene Bedeutung haben und Y für eine Abgangsgruppe steht.

Als inerte organische Lösungsmittel für das erste Verfahren kommen in Betracht: Äther wie Diäthyläther, Tetrahydrofuran, Dimethoxyäthan oder Dioxan und Kohlenwasserstoffe wie Hexan, Cyclohexan, Benzol, Toluol, Xylol oder Benzinfraktionen wie Petroläther oder Ligroin.

Als inerte organische Lösungsmittel für die beiden letztgenannten Verfahren kommen polare aprotische Lösungsmittel in Frage. Beispiele dafür sind Ketone wie Aceton oder Äthylmethylketon; Säureamide wie N,N-Dimethylformamid, Hexamethylphosphorsäuretriamid oder N-Methylpyrrolidon; Acetonitril oder Dimethylsulfoxid.

Beispiele für Basen sind im ersten Verfahren tertiäre Amine wie Triäthylamin, Pyridin oder 4-Dimethylaminopyridin; Carbonate wie Natrium- und Kaliumcarbonat; Hydrogencarbonate wie Natrium- oder Kaliumhydrogencarbonat; und Oxide wie Calcium- oder Magnesiumoxid.

Als Basen für die beiden letzten Verfahren können verwendet werden: Alkoholate wie Natriummethylat, Natriumäthylat, Kaliumäthylat oder Kalium-tert.-butylat; Hydride wie Natrium- oder Calciumhydrid; Hydroxide wie Natrium- oder Kaliumhydroxid; Carbonate wie Natrium- oder Kaliumcarbonat.

Geeignete Abgangsgruppen sind z.B. Halogen, vorzugsweise Chlor, Brom und Jod, insbesondere Brom und Jod; oder Sulfonsäurereste wie die von Toluolsulfonsäure, 4-Brombenzolsulfonsäure, Methansulfonsäure oder Trifluormethansulfonsäure.

Die Reaktionstemperaturen liegen bei allen drei Verfahren zwischen −20° und 170 °C, vorzugsweise zwischen 10 °C und dem Siedepunkt des Reaktionsgemisches.

Die Ausgangsverbindungen der Formeln III, IV, V, VI und VII sind bekannt und zum Teil im Handel erhältlich oder analog zu bekannten Verfahren leicht herstellbar.

Die Ausgangsverbindungen der Formel II sind über folgende Reaktionssequenz erhältlich:

Man hydrolysiert 2- [4- (6-Halogen-chinoxalinyl -2- oxy) -phenoxy] -propionsäurealkylester zu den freien Säuren und überführt diese nach an sich bekannten Verfahren, z.B. durch Umsetzung mit $SOCl_2$, $SOBr_2$, $POCl_3$, $PCl_3$, $PBr_3$, $PCl_5$, in die Säurehalogenide der Formel II.

Die Verbindungen der Formel I haben selektive Herbizideigenschaften. Sie eignen sich besonders zur Bekämpfung von monokotylen Unkräutern in dikotylen Kulturen wie Soja, Zuckerrübe, Baumwolle und verschiedenen Gemüsearten. Die Wirkstoffe schädigen dabei die dikotylen Kulturpflanzen nicht oder erst bei höheren Aufwandmengen. Die Wirkstoffe werden bei der Anwendung direkt auf die Pflanzen oder deren Samen oder auf den Lebensraum der Pflanzen in einer wirksamen Menge appliziert.

Darüberhinaus besitzen die Wirkstoffe der Formel I pflanzenwuchsregulierende Eigenschaften. Damit ist es möglich, das Wachstum der Pflanzen positiv zu beeinflussen. Solche Einflüsse können unter den folgenden Begriffen zusammengefasst werden: Wuchshemmung z.B. bei Gräsern in Parkanlagen oder an Böschungen; Wuchshemmung z.B. bei Getreiden zur Erhöhung der Widerstandskraft gegen Witterungseinflüsse; Wuchshemmung z.B. bei Unkräutern damit sie nicht zur Konkurrenz für Nutzpflanzen werden; Keimhemmung z.B. bei Lagerkartoffeln oder Saatgut; Förderung z.B. von Blüten- oder Fruchtansätzen bei Früchten oder Zierblumen; Reifebeschleunigung z.B. bei Früchten zur Vereinheitlichung des Erntezeitpunktes; Förderung des Wurzelwachstums z.B. bei Getreide oder Gemüse zur Verbesserung der Standfestigkeit und/oder der Nahrungsaufnahme der Pflanzen; oder Unterdrückung von Seitentrieben oder Blütenständen z.B. bei Tabak zur Erhöhung der Anzahl der Blätter.

Die erfindungsgemässen Wirkstoffe der Formel I sind in besonderer Weise dazu geeignet, das Wurzelwachstum zu fördern. Insbesondere wird das Wurzelwachstum von Getreidepflanzen wie Roggen, Gerste, Weizen und Mais stimuliert, wenn sie mit geringen Mengen der Wirkstoffe behandelt werden. Die Aufwandmengen liegen dabei generell unter denen, die einen herbiziden Effekt bewirken. Als vorteilhaft hat es sich weiter erwiesen die Wirkstoffe zur Wuchswachstumsförderung direkt nach der Einsaat auf den Boden zu applizieren oder aber das Saatgut vor der Aussaat mit den Wirkstoffen zu behandeln (Saatbeize).

Die Erfindung betrifft auch herbizide und pflanzenwuchsregulierende Mittel, welche einen neuen Wirkstoff der Formel I enthalten, sowie Verfahren zur pre- und post-emergenten Unkrautbekämpfung, insbesondere von monokotylen Ungräsern und Verfahren zur Beeinflussung des Pflanzenwachstums, insbesondere zur Förderung des Wurzelwachstums.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl -2- pyrrolidon, Dimethylsulfoxid oder Dimethylformamid, sowie gegebenenfalls epoxidierte Pflanzenöle wie epoxidiertes Kokusnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden.

Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol- (4–14) -Äthylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im(aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich

vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi (2-chloräthyl) äthylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corp., Ridgewood, New Jersey, 1979.

Sisley and Wood, «Encyclopedia of Surface Active Agents», Chemical Publishing Co., Inc. New York, 1964.

Die pestiziden Zubereitungen enthalten in der Regel 0,1 bis 99%, insbesondere 0,1 bis 95%, Wirkstoff der Formel I, 1 bis 99% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

| | |
|---|---|
| Lösungen | |
| Aktiver Wirkstoff: | 5 bis 95%, vorzugsweise 10 bis 80% |
| Lösungsmittel: | 95 bis 5%, vorzugsweise 90 bis 0% |
| oberflächenaktives Mittel: | 1 bis 30%, vorzugsweise 2 bis 20%. |
| Emulgierbare Konzentrate: | |
| Aktiver Wirkstoff: | 10 bis 50%, bevorzugt 10 bis 40% |
| oberflächenaktives Mittel: | 5 bis 30%, vorzugsweise 10 bis 20% |
| flüssiges Trägermittel: | 20 bis 95%, vorzugsweise 40 bis 80%. |
| Stäube | |
| Aktiver Wirkstoff: | 0,5 bis 10%, vorzugsweise 2 bis 8% |
| festes Trägermittel: | 99,5 bis 90%, vorzugsweise 98 bis 92%. |
| Suspensions-Konzentrate | |
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |
| Benetzbare Pulver | |
| Aktiver Wirkstoff: | 5 bis 90%, vorzugsweise 10 bis 80% und insbesondere 20 bis 60% |
| oberflächenaktives Mittel: | 0,5 bis 20%, vorzugsweise 1 bis 15% |
| festes Trägermittel: | 5 bis 90%, vorzugsweise 30 bis 70%. |
| Granulate | |
| Aktiver Wirkstoff: | 0,5 bis 30%, vorzugsweise 3 bis 15% |
| festes Trägermittel: | 99,5 bis 70%, vorzugsweise 97 bis 85% |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,1 bis 10 kg AS/ha, vorzugsweise 0,25 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele

Beispiel 1

a) Herstellung eines Zwischenproduktes

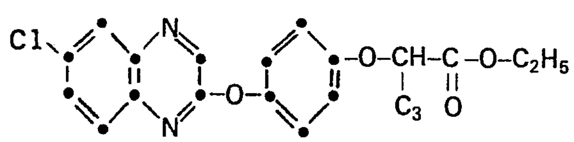

2- [4- (6-Chlor-chinoxalinyl -2- oxy)- phenoxy] - propionsäure-äthylester

24 g 2,6-Dichlorchinoxalin, 28 g 2- (4-Hydroxyphenoxy)- propionsäureäthylester und 24,8 g Kaliumcarbonat werden in 400 ml Äthylmethylketon für 12 Stunden unter Rückfluss erhitzt. Nach Abtrennen der entstandenen Salze wird die Lösung eingedampft und der Rückstand aus Diäthyläther kristallisiert. Man erhält 40 g 2- [4- (6-Chlorchinoxalinyl -2- oxy) -phenoxy] -propionsäure-äthylester. Smp. 90–93°.

b) Herstellung eines weiteren Zwischenproduktes

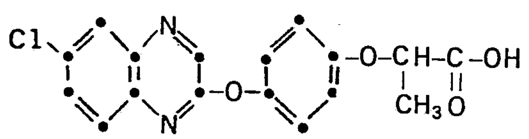

2- [4- (6-Chlor-chinoxalinyl -2- oxy) -phenoxy] - propionsäure

37,3 g 2- [4- (6-Chlor-chinoxalinyl -2- oxy)-phenoxy] -propionsäure-äthylester werden in einem Gemisch von 50 ml Äthanol und 70 ml wässriger 2 N Natriumhydroxidlösung für 2 Stunden am Rückfluss gekocht. Nach Filtrieren und Einengen des Reaktionsgemisches wird das Filtrat mit 32%iger Salzsäure angesäuert und mit Chloroform extrahiert. Eindampfen der Chloroformphase ergibt 26,8 g 2- [4- (6-Chlor-chinoxalinyl -2- oxy) -phenoxy] -propionsäure. Smp. 131–133°.

c) Herstellung eines weiteren Zwischenproduktes

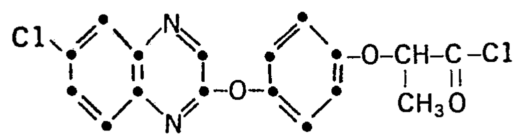

2- [4- (6-Chlor-chinoxalinyl -2- oxy) -phenoxy] - propionsäurechlorid

17,2 g 2- [4- (6-Chlor-chinoxalinyl -2- oxy) -phenoxy] -propionsäure werden mit 10 ml Thionylchlorid für 8 Stunden auf 60° erwärmt. Dann wird das überschüssige Thionylchlorid im Vakuum abgezogen, der Rückstand zur Vertreibung der restlichen Spuren von Thionylchlorid mit Toluol aufgenommen und erneut eingedampft. Das rohe ölige 2- [4- (6-Chlorchinoxalinyl -2- oxy)-phenoxy] -propionsäurechlorid kann direkt zur Herstellung von entsprechenden Estern eingesetzt werden.

d) Herstellung eines Endproduktes

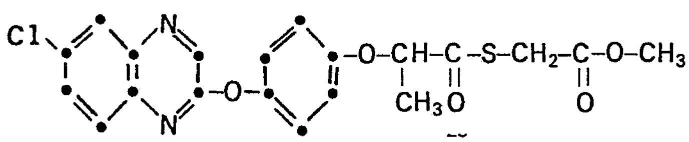 (Verb. Nr. 3)

2- [4- (6-Chlor-chinoxalinyl -2- oxy)- phenoxy]- thiopropionsäure-methoxy- carbonylmethylester

Zur Lösung von 3,6 g 2- [4- (6-Chlor-chinoxalinyl -2- oxy) -phenoxy] -propionsäurechlorid in 50 ml Toluol werden nacheinander 1,2 g Thioglykolsäuremethylester und 1,2 g Triäthylamin zugesetzt. Nachdem die Reaktionsmischung für 2 Stunden gerührt worden ist, wird der Niederschlag abgetrennt und das Filtrat mit 5%iger Salzsäure gewaschen, getrocknet und eingedampft. Man erhält 2,6 g 2- [4- (6-Chlor-chinoxalinyl -2- oxy) -phenoxy] -thiopropionsäure-methoxycarbonylmethylester als braunes Harz, das beim Verreiben mit Äther kristallisiert. Smp. 86–88 °C.

In der folgenden Tabelle 1 sind die nach dem Herstellungsbeispiel erhaltenen und analog herzustellenden Verbindungen der Formel I aufgeführt.

Tabelle 1: Verbindungen der Formel I

| Nr. | X | Z | A | R | phys.Daten |
|---|---|---|---|---|---|
| 1 | Cl | S | $-CH_2-CH_2-$ | $CH_3$ | |
| 2 | Cl | O | $-CH_2-CH_2-$ | $C_2H_5$ | |
| 3 | Cl | S | $-CH_2-$ | $CH_3$ | Smp. 86–88 °C |
| 4 | Cl | O | $-CH_2-$ | $CH_3$ | |
| 5 | Cl | 0 | $-CH(CH_3)-$ | $n-C_4H_9$ | $n_D^{30}$: 1,5570 |
| 6 | Cl | S | $-CH(CH_3)-$ | $CH_3$ | |
| 7 | Cl | O | $-CH(C_2H_5)-$ | $C_2H_5$ | $n_D^{25}$: 1,5675 |
| 8 | F | O | $-CH_2-$ | $C_2H_5$ | Smp. 88–90 °C |
| 9 | F | S | $-CH_2-$ | $CH_3$ | Smp. 97–99 °C |
| 10 | F | O | $-CH(CH_3)-$ | $CH_3$ | $n_D^{30}$: 1,5540 |
| 11 | F | S | $-CH(CH_3)-$ | $CH_3$ | |
| 12 | Cl | S | $-CH_2-$ | $C_2H_5$ | Smp. 84–86 °C |
| 13 | F | S | $-CH_2-$ | $C_2H_5$ | Smp. 92–95 °C |
| 14 | F | S | $-CH_2-$ | $n-C_4H_9$ | $n_D^{25}$: 1,5690 |

Formulierungsbeispiele

Beispiel 2

Formulierungsbeispiele für flüssige Wirkstoffe der Formel I
(% = Gewichtsprozent)

| a) Emulsionskonzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | – | – |
| Tributylphenoyl-polyäthylenglykoläther (30 Mol AeO) | – | 12% | 4,2% |
| Cyclohexanon | – | 15% | 20% |
| Xylolgemisch | 70% | 25% | 20% |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff | 80% | 10% | 5% | 95% |
| Äthylenglykol-monomethyl-äther | 20% | – | – | – |
| Polyäthylenglykol M G 400 | – | 70% | – | – |
| N-Methyl-2-pyrrolidon | – | 20% | – | – |
| Epoxidiertes Kokosnussöl | – | – | 1% | 5% |
| Benzin (Siedegrenzen 160–190 °C) | – | – | 94% | – |

Die Lösungen sind zur Anwendung in Form kleinster Tropfen geeignet.

| c) Granulate | a) | b) |
|---|---|---|
| Wirkstoff | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff | 2% | 5% |
| Hochdisperse Kieselsäure | 1% | 5% |
| Talkum | 97% | – |
| Kaolin | – | 90% |

Durch inniges Vermischen der Trägerstoffe mit dem Wirkstoff erhält man gebrauchsfertige Stäubemittel.

Beispiel 3

Formulierungsbeispiele für feste Wirkstoffe der Formel I
(% = Gewichtsprozent)

| a) Spritzpulver | a) | b) |
|---|---|---|
| Wirkstoff | 20% | 60% |
| Na-Ligninsulfonat | 5% | 5% |
| Na-Laurylsulfat | 3% | – |
| Na-Diisobutylnaphthalinsulfonat | – | 6% |
| Octylphenolpolyäthylenglykoläther 7–8 Mol AeO) | – | 2% |
| Hochdisperse Kieselsäure | 5% | 27% |
| Kaolin | 67% | – |

Der Wirkstoff wird mit den Zusatzstoffen gut vermischt und in einer geeigneten Mühle gut vermahlen. Man erhält Spritzpulver, die sich mit Wasser zur Suspensionen jeder gewünschten Konzentration verdünnen lassen.

| b) Emulsions-Konzentrat | |
|---|---|
| Wirkstoff | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 5% | 8% |
| Talkum | 95% | – |
| Kaolin | – | 92% |

Man erhält anwendungsfertige Stäubemittel, indem der Wirkstoff mit dem Träger vermischt und auf einer geeigneten Mühle vermahlen wird.

| d) Extruder Granulat | |
|---|---|
| Wirkstoff | 10% |
| Na-Ligninsulfonat | 2% |

| | |
|---|---|
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2% |
| Silikonöl in Form einer 75%igen wässrigen Emulsion | 0,8% |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel 4: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat, resp. aus einem 25%igen Spritzpulver mit Wirkstoffen, die wegen ungenügender Löslichkeit nicht als Emulsionskonzentrat hergestellt werden können, behandelt. Die Saatschalen werden im Gewächshaus bei 22–25 °C und 50–70% relativer Luftfeuchtigkeit gehalten und der Versuch nach 3 Wochen ausgewertet.

Die Verbindungen der Formel I zeigten in diesem Versuch eine ausgezeichnete Herbizidwirkung gegen monokotyle und einige dikotyle Unkräuter. Dabei werden dikotyle Kulturpflanzen nicht oder bei höheren Aufwandmengen nur geringfügig in ihrem Wachstum beeinflusst.

Pre-emergente Herbizidwirkung:

| Verb. kg AS/ha | Verb. Nr. 3 | | Verb. Nr. 5 | | Verb. Nr. 7 | |
|---|---|---|---|---|---|---|
| Testpflanze | 4 kg | 2 kg | 4 kg | 2 kg | 4 kg | 2 kg |
| Alopecurus myos. | 2 | 2 | 1 | 1 | 1 | 1 |
| Avena fatua | 6 | – | 2 | – | 5 | – |
| Echinochloa c.g. | 1 | 1 | 1 | 1 | 1 | 1 |
| Rottboellia ex. | 1 | 2 | 1 | 1 | 1 | 1 |
| Setaria ital. | 1 | – | 1 | – | 1 | – |
| Soja | 9 | 9 | 9 | 9 | 9 | 9 |
| Baumwolle | 9 | 9 | 9 | 9 | 9 | 9 |
| Zuckerrübe | 9 | 9 | 9 | 9 | 9 | 9 |
| Sinapis alba | 9 | – | 6 | – | 9 | – |
| Stellaria med. | 7 | – | 9 | – | 9 | – |

Beispiel 5: Post-emergente Herbizid-Wirkung (Kontaktherbizid)

Ein oder mehrere Unkräuter und Kulturpflanzen, sowohl monokotyle wie dikotyle wurden nach dem Auflaufen (im 4- bis 6-Blattstadium) mit einer wässrigen Wirkstoffdispersion in verschiedenen Dosierungen auf die Pflanzen gespritzt und diese bei 23–26 °C und 45–60% relativer Luftfeuchtigkeit gehalten. 15 Tage nach Behandlung wird der Versuch ausgewertet.

Auch in diesem Versuch erwiesen sich die Verbindungen der Formel I als hervorragende selektive Herbizide gegen monokotyle und auch einige dikotyle Unkräuter. Dikotyle Kulturpflanzen wurden dabei nicht oder erst bei höheren Aufwandmengen geschädigt.

Post-emergente Herbizidwirkung:

| Verb. kg AS/ha | Verb. Nr. 3 | | Verb. Nr. 5 | | Verb. Nr. 7 | |
|---|---|---|---|---|---|---|
| Testpflanze | 1 kg | 0,5 kg | 1 kg | 0,5 kg | 1 kg | 0,5 kg |
| Avena fatua | 2 | 7 | 1 | 1 | 1 | 1 |
| Alopecurus myos. | 2 | 2 | 1 | 1 | 1 | 1 |
| Echinochloa c.g. | 1 | 1 | 1 | 1 | 1 | 1 |
| Rottboellia ex. | 1 | 2 | 1 | 1 | 1 | 1 |
| Soja | 9 | 9 | 6 | 7 | 7 | 8 |
| Baumwolle | 9 | 9 | 8 | 9 | 9 | 9 |
| Zuckerrübe | 9 | 9 | 7 | 9 | 8 | 9 |

Beispiel 6: Förderung des Wurzelwachstums

a) Saatbeize: Die Wirkstoffe der Formel I werden in Aufwandmengen von 13 mg Aktivsubstanz/kg Saatgut als wässrige Emulsion auf das Saatgut gesprüht. Je 10 der so behandelten Samen werden in flache mit Erde gefüllte Schalen gesät und in Klimakammern unter kontrollierten Bedingungen gehalten. Nach 10 Tagen, nachdem die Samen gekeimt haben, werden die Keimlinge vorsichtig aus der Erde herausgewaschen. Zur Auswertung des Versuchs werden die Wurzellänge und das Wurzelgewicht im Vergleich zu Keimlingen aus ungebeizten Samen bestimmt.

Resultate: (in % im Vergleich zur unbehandelten Kontrolle)
Testpflanze: Weizen
Wirkstoff: Verbindung Nr. 3

| Wurzellänge | | Wurzelgewicht | |
|---|---|---|---|
| cm | % zur Kontrolle | mg | % zur Kontrolle |
| 12,0 | 145 | 48 | 41 |

b) Bodenbehandlung: Sofort nach der Aussaat von jeweils 10 Samen in flache mit Erde gefüllte Schalen werden die Wirkstoffe als wässrige Emulsionen in Aufwandmengen von 0,3 und 1,0 kg Aktivsubstanz/Hektar auf die Oberfläche der Saatschalen gesprüht. Danach werden die Schalen unter kontrollierten Bedingungen in Klimakammern gehalten. Nach 10 Tagen erfolgt die Auswertung des Versuchs nach den gleichen Kriterien wie im Versuch a).

Resultate: (in % im Vergleich zur unbehandelten Kontrolle)
Testpflanze: Weizen
Wirkstoff: Verbindung Nr. 3

| Aufwandmenge | Wurzellänge | | Wurzelgewicht | |
|---|---|---|---|---|
| | cm | % zur Kontrolle | mg | % zur Kontrolle |
| 0,3 kg AS/ha | 13,0 | 163 | 124 | 115 |
| 1,0 kg AS/ha | 10,0 | 125 | 98 | 91 |

**Patentansprüche für die Vertragsstaaten CH, DE, FR, GB, IT, LI**

1. 2- [4- (6-Halogen-chinoxalinyl -2- oxy) -phenoxy] -propionsäureester der allgemeinen Formel I,

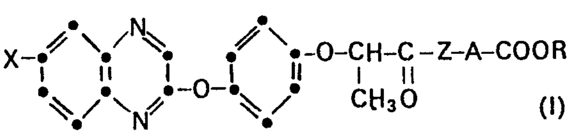

(I)

worin X Fluor, Chlor oder Brom, Z Sauerstoff oder Schwefel, A eine gegebenenfalls durch Methyl oder Äthyl substituierte $C_1$-$C_4$-Alkylenbrücke und R $C_1$-$C_4$-Alkyl bedeuten, mit der Massgabe, dass die Gruppe –Z–A–COOR nicht für $-O-CH(CH_3)-COOC_2H_5$ steht.

2. Verbindungen der Formel I gemäss Anspruch 1, dadurch gekennzeichnet, dass X Fluor oder Chlor bedeutet.

3. Verbindungen der Formel I gemäss Anspruch 1, dadurch gekennzeichnet, dass A eine gegebenenfalls durch Methyl oder Äthyl substituierte $C_1$-$C_2$-Alkylenbrücke bildet, die insgesamt höchstens 3 Kohlenstoffatome enthält.

4. Verbindungen der Formel I gemäss den Ansprüchen 2 und 3, dadurch gekennzeichnet, dass X Fluor oder Chlor und A eine gegebenenfalls durch Methyl oder Äthyl substituierte $C_1$-$C_2$-Alkylenbrücke bildet, die insgesamt höchstens 3 Kohlenstoffatome enthält.

5. 2- [4- (6-Chlorchinoxalinyl -2- oxy) -phenoxy] -thiopropionsäure- methoxycarbonylmethylester gemäss Anspruch 1.

6. 2- [4- (6-Chlorchinoxalinyl -2- oxy) -phenoxy] -propionsäure -1- (äthoxycarbonyl) -n-propylester gemäss Anspruch 1.

7. 2- [4- (6-Chlorchinoxalinyl -2- oxy) -phenoxy] -propionsäure -1- (n-butyloxycarbonyl)-äthylester gemäss Anspruch 1.

8. 2- [4- (6-Fluorchinoxalinyl -2- oxy) -phenoxy] -propionsäure- äthoxy- carbonylmethylester gemäss Anspruch 1.

9. 2- [4- (6-Fluorchinoxalinyl -2- oxy) -phenoxy] -thiopropionsäure- methoxycarbonylmethylester gemäss Anspruch 1.

10. 2- [4- (6-Fluorchinoxalinyl -2- oxy)-phenoxy] -propionsäure -1- (methoxycarbonyl)-äthylester gemäss Anspruch 1.

11. 2- [4- (6-Fluorchinoxalinyl -2- oxy)-phenoxy] -thiopropionsäure -n- butyloxycarbonylmethylester gemäss Anspruch 1.

12. Verfahren zur Herstellung der Verbindungen der Formel I gemäss Anspruch 1, dadurch gekennzeichnet, dass man

a) ein Säurehalogenid der Formel II,

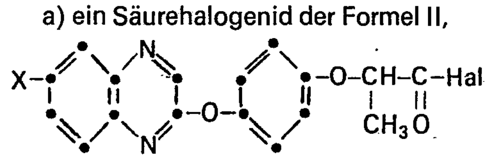

(II)

worin X die unter Formel I angegebene Bedeutung hat und Hal für Chlor oder Brom steht, gegebenenfalls in einem inerten Lösungsmittel und gegebenenfalls in Gegenwart einer Base mit einem Alkancarbonsäureester der Formel III

H–Z–A–COOR (III)

umsetzt, worin A, R und Z die unter Formel I gegebene Bedeutung haben, oder

b) ein Chinoxalinylchlorid der Formel IV,

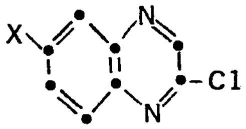

(IV)

worin X die unter Formel I angegebene Bedeutung hat, in einem inerten Lösungsmittel, gegebenenfalls in Gegenwart einer Base, mit einem Phenoxypropionsäureester der Formel V

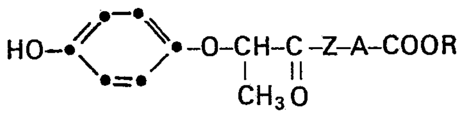

(V)

umsetzt, worin A, R und Z die unter Formel I angegebene Bedeutung haben, oder

c) ein Chinoxalinyl -2- oxyphenol der Formel VI,

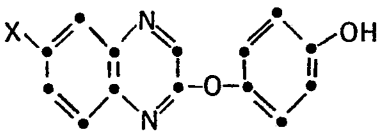

(VI)

worin X die unter Formel I angegebene Bedeutung hat, in einem inerten Lösungsmittel, gegebenenfalls in Gegenwart einer Base, mit einem Propionsäureester der Formel VII

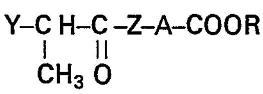

(VII)

umsetzt, worin A, R und Z die unter Formel I angegebene Bedeutung haben und Y für eine Abgangsgruppe steht.

13. Herbizides und pflanzenwuchsregulierendes Mittel, dadurch gekennzeichnet, dass es neben Träger- und/oder anderen Zuschlagstoffen als Wirkstoff mindestens eine Verbindung gemäss Anspruch 1 enthält.

14. Verfahren zur Bekämpfung unerwünschten Pflanzenwuchses, dadurch gekennzeichnet, dass man einen Wirkstoff der Formel I gemäss Anspruch 1, in einer wirksamen Menge auf die unerwünschten Pflanzen oder deren Lebensraum einwirken lässt.

15. Verfahren gemäss Anspruch 14 zur selektiven Bekämpfung von monokotylen Unkräutern in Kulturpflanzenbeständen.

16. Verwendung gemäss einem der Ansprüche 14 oder 15, dadurch gekennzeichnet, dass der Wirkstoff postemergent angewendet wird.

17. Verfahren zur Beeinflussung des Pflanzenwuchses, dadurch gekennzeichnet, dass man einen Wirkstoff der Formel I gemäss Anspruch 1, in einer wirksamen Menge auf die zu beeinflussenden Pflanzen oder deren Lebensraum einwirken lässt.

18. Verfahren gemäss Anspruch 17, zur Stimulation des Wurzelwachstums bei Getreide.

19. Verfahren gemäss einem der Ansprüche 17 oder 18, dadurch gekennzeichnet, dass sie preemergent erfolgt.

20. Verfahren gemäss Anspruch 18, dadurch gekennzeichnet, dass das Saatgut vor der Aussaat mit den Wirkstoffen behandelt wird.

**Patentansprüche für die Vertragsstaaten BE, NL**

1. 2- [4- (6-Halogen-chinoxalinyl -2- oxy)-phenoxy] -propionsäureester der allgemeinen Formel I,

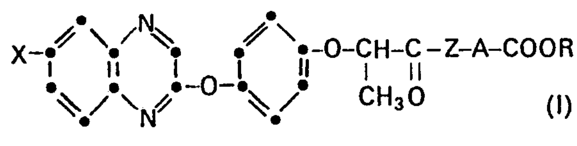

(I)

worin X Fluor, Chlor oder Brom, Z Sauerstoff oder Schwefel, A eine gegebenenfalls durch Methyl oder Äthyl substituierte $C_1$-$C_4$-Alkylenbrücke und R $C_1$-$C_4$-Alkyl bedeuten.

2. Verbindungen der Formel I gemäss Anspruch 1, dadurch gekennzeichnet, dass X Fluor oder Chlor bedeutet.

3. Verbindungen der Formel I gemäss Anspruch 1, dadurch gekennzeichnet, dass A eine gegebenenfalls durch Methyl oder Äthyl substituierte $C_1$-$C_2$-Alkylenbrücke bildet, die insgesamt höchstens 3 Kohlenstoffatome enthält.

4. Verbindungen der Formel I gemäss den Ansprüchen 2 und 3, dadurch gekennzeichnet, dass X Fluor oder Chlor und A eine gegebenenfalls durch Methyl oder Äthyl substituierte $C_1$-$C_2$-Alkylenbrücke bildet, die insgesamt höchstens 3 Kohlenstoffatome enthält.

5. 2- [4- (6-Chlorchinoxalinyl -2- oxy) -phenoxy] -thiopropionsäure- methoxycarbonylmethylester gemäss Anspruch 1.

6. 2- [4- (6-Chlorchinoxalinyl -2- oxy) -phenoxy] -propionsäure -1- (äthoxycarbonyl) -n-propylester gemäss Anspruch 1.

7. 2- [4- (6-Chlorchinoxalinyl -2- oxy) -phenoxy] -propionsäure -1- (n-butyloxycarbonyl)-äthylester gemäss Anspruch 1.

8. 2- [4- (6-Fluorchinoxalinyl -2- oxy) -phenoxy] -propionsäure-äthoxy- carbonylmethylester gemäss Anspruch 1.

9. 2- [4- (6-Fluorchinoxalinyl -2- oxy) -phenoxy] -thiopropionsäure- methoxycarbonylmethylester gemäss Anspruch 1.

10. 2- [4- (6-Fluorchinoxalinyl -2- oxy)-phenoxy] -propionsäure -1- (methoxycarbonyl)-äthylester gemäss Anspruch 1.

11. 2- [4- (6-Fluorchinoxalinyl -2- oxy)-phenoxy] -thiopropionsäure -n- butyloxycarbonylmethylester gemäss Anspruch 1.

12. Verfahren zur Herstellung der Verbindungen der Formel I gemäss Anspruch 1, dadurch gekennzeichnet, dass man

a) ein Säurehalogenid der Formel II,

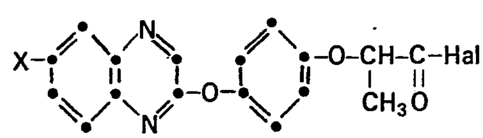

(II)

worin X die unter Formel I angegebene Bedeutung hat und Hal für Chlor oder Brom steht, gegebenenfalls in einem inerten Lösungsmittel und gegebenenfalls in Gegenwart einer Base mit einem Alkancarbonsäureester der Formel III

H-Z-A-COOR (III)

umsetzt, worin A, R und Z die unter Formel I gegebene Bedeutung haben, oder

b) ein Chinoxalinylchlorid der Formel IV,

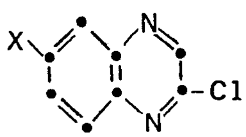

(IV)

worin X die unter Formel I angegebene Bedeutung hat, in einem inerten Lösungsmittel, gegebenenfalls in Gegenwart einer Base, mit einem Phenoxypropionsäureester der Formel V

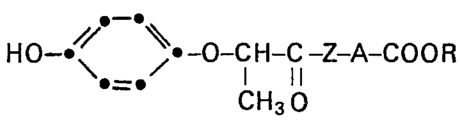

(V)

umsetzt, worin A, R und Z die unter Formel I angegebene Bedeutung haben, oder

c) ein Chinoxalinyl -2- oxyphenol der Formel VI,

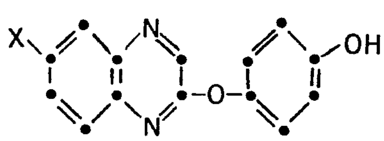

(VI)

worin X die unter Formel I angegebene Bedeutung hat, in einem inerten Lösungsmittel, gegebenenfalls in Gegenwart einer Base, mit einem Propionsäureester der Formel VII

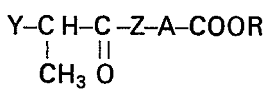

(VII)

umsetzt, worin A, R und Z die unter Formel I angegebene Bedeutung haben und Y für eine Abgangsgruppe steht.

13. Herbizides und pflanzenwuchsregulierendes Mittel, dadurch gekennzeichnet, dass es neben Träger- und/oder anderen Zuschlagstoffen als Wirkstoff mindestens eine Verbindung gemäss Anspruch 1 enthält.

14. Verfahren zur Bekämpfung unerwünschten Pflanzenwuchses, dadurch gekennzeichnet, dass man einen Wirkstoff der Formel I gemäss Anspruch 1, in einer wirksamen Menge auf die unerwünschten Pflanzen oder deren Lebensraum einwirken lässt.

15. Verfahren gemäss Anspruch 14 zur selektiven Bekämpfung von monokotylen Unkräutern in Kulturpflanzenbeständen.

16. Verwendung gemäss einem der Ansprüche 14 oder 15, dadurch gekennzeichnet, dass der Wirkstoff postemergent angewendet wird.

17. Verfahren zur Beeinflussung des Pflanzenwuchses, dadurch gekennzeichnet, dass man einen Wirkstoff der Formel I gemäss Anspruch 1, in einer wirksamen Menge auf die zu beeinflussenden Pflanzen oder deren Lebensraum einwirken lässt.

18. Verfahren gemäss Anspruch 17, zur Stimulation des Wurzelwachstums bei Getreide.

19. Verfahren gemäss einem der Ansprüche 17 oder 18, dadurch gekennzeichnet, dass sie preemergent erfolgt.

20. Verfahren gemäss Anspruch 18, dadurch gekennzeichnet, dass das Saatgut vor der Aussaat mit den Wirkstoffen behandelt wird.

**Patentansprüche für den Vertragsstaat AT**

1. Herbizides und pflanzenwuchsregulierendes Mittel, dadurch gekennzeichnet, dass es neben Träger- und/oder anderen Zuschlagstoffen als Wirkstoff mindestens einen 2- [4- (6-Halogenchinoxalinyl -2- oxy) -phenoxy] -propionsäureester der allgemeinen Formel I,

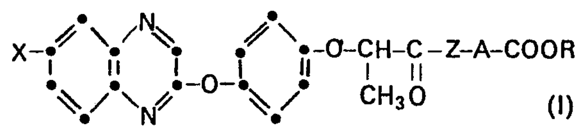

(I)

worin X Fluor, Chlor oder Brom Z Sauerstoff oder Schwefel, A eine gegebenenfalls durch Methyl oder Äthyl substituierte $C_1$-$C_4$-Alkylenbrücke und R $C_1$-$C_4$-Alkyl bedeuten, enthält.

2. Mittel gemäss Anspruch 1, dadurch gekennzeichnet, dass im Wirkstoff der Formel I X Fluor oder Chlor bedeutet.

3. Mittel gemäss Anspruch 1, dadurch gekennzeichnet, dass im Wirkstoff der Formel I A eine gegebenenfalls durch Methyl oder Äthyl substituierte $C_1$-$C_2$-Alkylenbrücke bildet, die insgesamt höchstens 3 Kohlenstoffatome enthält.

4. Mittel gemäss den Ansprüchen 2 und 3, dadurch gekennzeichnet, dass X Fluor oder Chlor und A eine gegebenenfalls durch Methyl oder Äthyl substituierte $C_1$-$C_2$-Alkylenbrücke bildet, die insgesamt 3 Kohlenstoffatome enthält.

5. Mittel gemäss Anspruch 1, dadurch gekennzeichnet, dass es als Wirkstoff 2- [4- (6-Chlorchinoxalinyl -2- oxy) -phenoxy]- thiopropionsäure- methoxycarbonylmethylester enthält.

6. Mittel gemäss Anspruch 1, dadurch gekennzeichnet, dass es als Wirkstoff 2- [4- (6-Chlorchinoxalinyl -2- oxy) -phenoxy] -propionsäure - 1- (äthoxy-carbonyl) -n- propylester enthält.

7. Mittel gemäss Anspruch 1, dadurch gekennzeichnet, dass es als Wirkstoff 2- [4- (6-Chlorchinoxalinyl -2- oxy) -phenoxy] -propionsäure-1- (n-butyloxycarbonyl) -äthylester enthält.

8. Mittel gemäss Anspruch 1, dadurch gekennzeichnet, dass es als Wirkstoff 2- [4- (6-Fluorchinoxalinyl -2- oxy) -phenoxy] -propionsäureäthoxycarbonylmethylester enthält.

9. Mittel gemäss Anspruch 1, dadurch gekennzeichnet, dass es als Wirkstoff 2- [4- (6-Fluorchinoxalinyl -2- oxy) -phenoxy]- thiopropionsäure-methoxycarbonylmethylester enthält.

10. Mittel gemäss Anspruch 1, dadurch gekennzeichnet, dass es als Wirkstoff 2- [4- (6-Fluorchinoxalinyl -2- oxy) -phenoxy] -propionsäure -1- (methoxycarbonyl) -äthylester enthält.

11. Mittel gemäss Anspruch 1, dadurch gekennzeichnet, dass es als Wirkstoff 2- [4- (6-Fluorchinoxalinyl -2- oxy) -phenoxy] -thiopropionsäure -n- butyloxycarbonylmethylester enthält.

12. Verfahren zur Herstellung der Wirkstoffe der Formel I, dadurch gekennzeichnet, dass man

a) ein Säurehalogenid der Formel II,

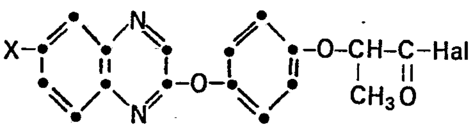

(II)

worin X die unter Formel I angegebene Bedeutung hat und Hal für Chlor oder Brom steht, gegebenenfalls in einem inerten Lösungsmittel und gegebenenfalls in Gegenwart einer Base mit einem Alkancarbonsäureester der Formel III

H-Z-A-COOR (III)

umsetzt, worin A, R und Z die unter Formel I gegebene Bedeutung haben oder

b) ein Chinoxalinylchlorid der Formel IV,

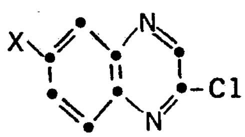

(IV)

worin X die unter Formel I angegebene Bedeutung hat in einem inerten Lösungsmittel, gegebenenfalls in Gegenwart einer Base, mit einem Phenoxypropionsäureester der Formel V

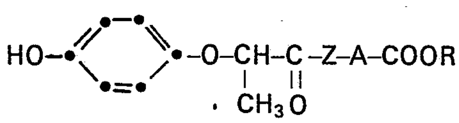

(V)

umsetzt, worin A, R und Z die unter Formel I angegebene Bedeutung haben oder

c) ein Chinoxalinyl -2- oxyphenol der Formel VI,

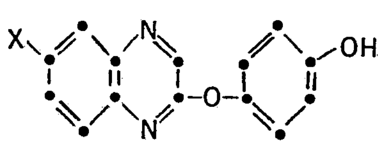

(VI)

worin X die unter Formel I angegebene Bedeutung hat, in einem inerten Lösungsmittel, gegebenenfalls in Gegenwart einer Base, mit einem Propionsäureester der Formel VII

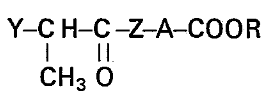

(VII)

umsetzt, worin A, R und Z die unter Formel I angegebene Bedeutung haben und Y für eine Abgangsgruppe steht.

13. Verfahren zur Bekämpfung unerwünschten Pflanzenwuchses, dadurch gekennzeichnet, dass man ein Mittel gemäss Anspruch 1, in einer wirksamen Menge auf die unerwünschten Pflanzen oder deren Lebensraum einwirken lässt.

14. Verfahren gemäss Anspruch 13 zur selektiven Bekämpfung von monokotylen Unkräutern in Kulturpflanzenbeständen.

15. Verfahren gemäss einem der Ansprüche 13 oder 14, dadurch gekennzeichnet, dass das Mittel postemergent angewendet wird.

16. Verfahren zur Beeinflussung des Pflanzenwuchses, dadurch gekennzeichnet, dass man ein Mittel gemäss Anspruch 1, in einer wirksamen Menge auf die zu beeinflussenden Pflanzen oder deren Lebensraum einwirken lässt.

17. Verfahren gemäss Anspruch 16, zur Stimulation des Wurzelwachstums bei Getreide.

18. Verfahren gemäss einem der Ansprüche 16 oder 17, dadurch gekennzeichnet, dass sie preemergent erfolgt.

19. Verfahren gemäss Anspruch 17, dadurch gekennzeichnet, dass das Saatgut vor der Aussaat mit dem Mittel behandelt wird.

**Revendications pour les Etats contractants: CH, DE, FR, GB, IT, LI**

1. Ester d'acide 2- [4- (6-halogène-quinoxalinyl -2- oxy) -phénoxy] -propionique de formule générale I

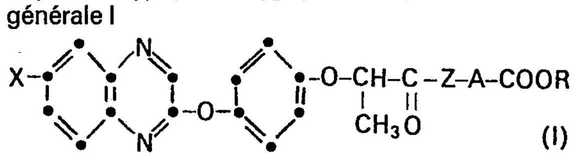

(I)

où X représente un fluor, un chlore ou un brome, Z représente un oxygène ou un soufre. A représente un pont alcoylène en C1 à C4 éventuellement substitué par un méthyle ou un éthyle et R représente un alcoyle en C1 à C4 avec la précision que le groupe –Z–A–COOR ne représente pas $-O-CH(CH_3)-COOC_2H_5$.

2. Composés de formule I selon la revendication 1, caractérisé en ce que X représente un fluor ou un chlore.

3. Composés de formule I selon la revendication 1, caractérisés en ce que A forme un pont alcoylène en C1 à C2 éventuellement substitué par un méthyle ou un éthyle, qui contient au total au plus 3 atomes de carbone.

4. Composés de formule I selon les revendications 2 et 3, caractérisés en ce que X représente un fluor ou un chlore et A forme un pont alcoylène en C1 à C2 éventuellement substitué par un méthyle ou un éthyle, qui contient au total au plus 3 atomes de carbone.

5. Méthoxycarbonylméthylester de l'acide 2- [4- (6-chloro-quinoxalinyl -2- oxy) -phénoxy] -thiopropionique, selon la revendication 1.

6. 1- (éthoxycarbonyl) -n- propylester de l'acide 2- [4- (6-chloro-quinoxalinyl -2- oxy) -phénoxy] -propionique selon la revendication 1.

7. 1- (n-butyloxycarbonyl) -éthylester de l'acide 2- [4- (6-chloro-quinoxalinyl -2- oxy) -phénoxy] -propionique selon la revendication 1.

8. Ethoxycarbonylméthylester de l'acide 2- [4- (6-fluoroquinoxalinyl -2- oxy) -phénoxy] -propionique, selon la revendication 1.

9. Méthoxycarbonylméthylester de l'acide 2- [4- (6-fluoroquinoxalinyl -2- oxy) -phénoxy]-thiopropionique, selon la revendication 1.

10. 1- (méthoxycarbonyl) -éthylester de l'acide 2- [4- (6-fluoroquinoxalinyl -2- oxy) -phénoxy]-propionique, selon la revendication 1.

11. n-butyloxycarbonylméthylester de l'acide 2- [4- (6-fluoroquinoxalinyl -2- oxy) -phénoxy]-thiopropionique, selon la revendication 1.

12. Procédé de préparation des composés de formule I selon la revendication 1, caractérisé en ce que

a) on fait réagir un halogénure d'acide de formule II

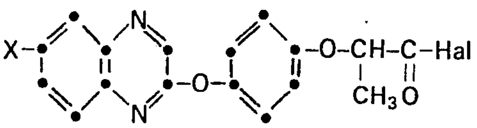

(II)

où X a la signification donnée pour la formule I et Hal représente un chlore ou un brome, éventuellement dans un solvant inerte et éventuellement en présence d'une base avec un ester d'acide alcanecarboxylique de formule III

H-Z-A-COOR (III)

où A, R et Z ont la signification donnée pour la formule I, ou

b) on fait réagir un chlorure de quinoxalinyle de formule IV

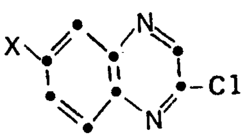

(IV)

où X a la signification donnée dans la formule I, dans un solvant inerte, éventuellement en présence d'une base, avec un ester d'acide phénoxypropionique de formule V

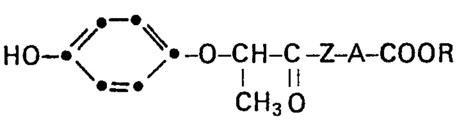

(V)

où A, R et Z ont la signification donnée pour la formule I, ou

c) on fait réagir un quinoxalinyl -2- oxyphénol de formule VI

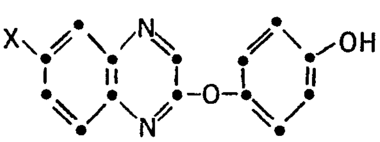

(VI)

où X a la signification donnée dans la formule I, dans un solvant inerte, éventuellement en présence d'une base, avec un ester d'acide propionique de formule VII

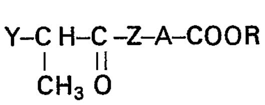

(VII)

où A, R et Z ont la signification donnée pour la formule I et Y représente un groupe sortant.

13. Agent herbicide et de régulation de la croissance des plantes, caractérisé en ce qu'il contient, outre des supports et/ou autres additifs, comme matière active au moins un composé selon la revendication 1.

14. Procédé pour combattre la croissance végétale indésirable, caractérisé en ce qu'on fait agir une matière active de formule I selon la revendication 1, en une quantité efficace sur les plantes indésirables ou leur biotope.

15. Procédé selon la revendication 14 de lutte sélective contre les mauvaises herbes monocotylédones dans les plantes cultivées.

16. Application selon l'une des revendications 14 et 15, caractérisée en ce qu'on applique la matière active en post-levée.

17. Procédé pour influencer la croissance des plantes, caractérisé en ce qu'on fait agir une matière active de formule I selon la revendication 1 en une quantité efficace sur les plantes à influencer ou leur biotope.

18. Procédé selon la revendication 17 pour stimuler la croissance des racines dans les céréales.

19. Procédé selon l'une des revendications 17 et 18, caractérisé en ce qu'il s'effectue en pré-levée.

20. Procédé selon la revendication 18, caractérisé en ce qu'on traite les semences avec les matières actives avant l'ensemencement.

**Revendications pour les Etats contractants: BE, NL**

1. Ester d'acide 2- [4- (6-halogène-quinoxalinyl -2- oxy) -phénoxy-propionique de formule générale I

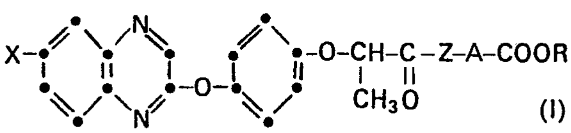

(I)

où X représente un fluor, un chlore ou un brome, Z représente un oxygène ou un soufre. A représente un pont alcoylène en C1 à C4 éventuellement substitué par un méthyle ou un éthyle et R représente un alcoyle en C1 à C4.

2. Composés de formule I selon la revendication 1, caractérisé en ce que X représente un fluor ou un chlore.

3. Composés de formule I selon la revendication 1, caractérisés en ce que A forme un pont alcoylène en C1 à C2 éventuellement substitué par un méthyle ou un éthyle, qui contient au total au plus 3 atomes de carbone.

4. Composés de formule I selon les revendications 2 et 3, caractérisés en ce que X représente un fluor ou un chlore et A forme un pont alcoylène en C1 à C2 éventuellement substitué par un méthyle ou un éthyle, qui contient au total au plus 3 atomes de carbone.

5. Méthoxycarbonylméthylester de l'acide 2- [4- (6-chloro-quinoxalinyl -2- oxy) -phénoxy]-thiopropionique, selon la revendication 1.

6. 1- (éthoxycarbonyl) -n- propylester de l'acide 2- [4- (6-chloro-quinoxalinyl -2- oxy) -phénoxy] -propionique, selon la revendication 1.

7. 1- (n-butyloxycarbonyl) -éthylester de l'acide 2- [4- (6-chloro-quinoxalinyl -2- oxy) -phénoxy] -propionique, selon la revendication 1.

8. Ethoxycarbonylméthylester de l'acide 2- [4- (6-fluoroquinoxalinyl -2- oxy) -phénoxy] -propionique, selon la revendication 1.

9. Méthoxycarbonylméthylester de l'acide 2- [4- (6- fluoroquinoxalinyl -2- oxy) -phénoxy]-thiopropionique, selon la revendication 1.

10. 1- (méthoxycarbonyl)- éthylester de l'acide 2- [4- (6-fluoroquinoxalinyl -2- oxy)- phénoxy]-propionique, selon la revendication 1.

11. n-butyloxycarbonylméthylester de l'acide 2- [4- (6-fluoroquinoxalinyl -2- oxy) -phénoxy]-thiopropionique, selon la revendication 1.

12. Procédé de préparation des composés de formule I selon la revendication 1, caractérisé en ce que

a) on fait réagir un halogénure d'acide de formule II

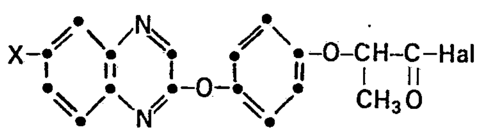

(II)

où X a la signification donnée pour la formule I et Hal représente un chlore ou un brome, éventuellement dans un solvant inerte et éventuellement en présence d'une base avec un ester d'acide alcanecarboxylique de formule III

H–Z–A–COOR (III)

où A, R et Z ont la signification donnée pour la formule I, ou

b) on fait réagir un chlorure de quinoxalinyle de formule IV

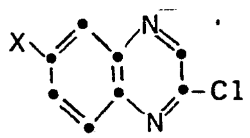

(IV)

où X a la signification donnée dans la formule I, dans un solvant inerte, éventuellement en présence d'une base, avec un ester d'acide phénoxypropionique de formule V

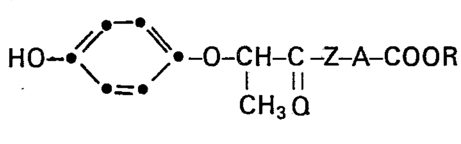

(V)

où A, R et Z ont la signification donnée pour la formule I, ou

c) on fait réagir un quinoxalinyl -2- oxyphénol de formule VI

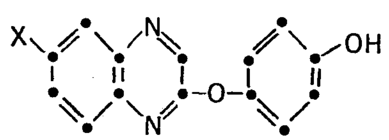

(VI)

où X a la signification donnée dans la formule I, dans un solvant inerte, éventuellement en présence d'une base, avec un ester d'acide propionique de formule VII

Y–CH–C–Z–A–COOR (VII)
  |   ||
 $CH_3$ O

où A, R et Z ont la signification donnée pour la formule I et Y représente un groupe sortant.

13. Agent herbicide et de régulation de la croissance des plantes, caractérisé en ce qu'il contient, outre des supports et/ou autres additifs, comme matière active au moins un composé selon la revendications 1.

14. Procédé pour combattre la croissance végétale indésirable, caractérisé en ce qu'on fait agir une matière active de formule I selon la revendication 1, en une quantité efficace sur les plantes indésirables ou leur biotope.

15. Procédé selon la revendication 14 de lutte sélective contre les mauvaises herbes monocotylédones dans les plantes cultivées.

16. Application selon l'une des revendications 14 et 15, caractérisée en ce qu'on applique la matière active en post-levée.

17. Procédé pour influencer la croissance des plantes, caractérisé en ce qu'on fait agir une matière active de formule I selon la revendication 1 en une quantité efficace sur les plantes à influencer ou leur biotope.

18. Procédé selon la revendication 17 pour stimuler la croissance des racines dans les céréales.

19. Procédé selon l'une des revendications 17 et 18, caractérisé en ce qu'il s'effectue en pré-levée.

20. Procédé selon la revendication 18, caractérisé en ce qu'on traite les semences avec les matières actives avant l'ensemencement.

**Revendications pour l'Etat contractant: AT**

1. Agent herbicide et de régulation de la croissance des plantes, caractérisé en ce qu'il contient, outre des supports et/ou autres additifs, comme matière active au moins un ester d'acide 2- [4- (6-halogène-quinoxalinyl -2- oxy) -phénoxy]-propionique de formule générale I

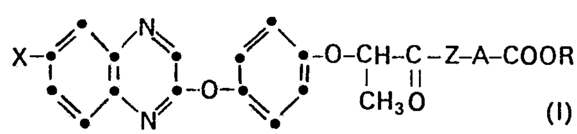

(I)

où X représente un fluor, un chlore ou un brome, Z représente un oxygène ou un soufre. A représente un pont alcoylène en C1 à C4 éventuellement substitué par un méthyle ou un éthyle et R représente un alcoyle en C1 à C4.

2. Agent selon la revendication 1, caractérisé en ce que dans la matière active de formule I X représente un fluor ou un chlore.

3. Agent selon la revendication 1, caractérisé en ce que dans la matière active de formule I A forme un pont alcoylène en C1 à C2 éventuellement substitué par un méthyle ou un éthyle, qui contient au total au plus 3 atomes de carbone.

4. Agent selon les revendications 2 et 3, caractérisé en ce que X représente un fluor ou un chlore et A forme un pont alcoylène éventuellement substitué par un méthyle ou un éthyle, qui contient au total 3 atomes de carbone.

5. Agent selon la revendication 1, caractérisé en ce qu'il contient comme matière active le Méthoxycarbonylméthylester de l'acide 2- [4- (6-chloro-quinoxalinyl -2- oxy) -phénoxy] -thiopropionique.

6. Agent selon la revendication 1, caractérisé en ce qu'il contient comme matière active le 1-[éthoxycarbonyl) -n- propylester de l'acide 2- [4-(6-chloro-quinoxalinyl -2- oxy) -phénoxy] -propionique.

7. Agent selon la revendication 1, caractérisé en ce qu'il contient comme matière active le 1- (n-butyloxycarbonyl) -éthylester de l'acide 2- [4-(6-chloro-quinoxalinyl -2- oxy) -phénoxy] -propionique.

8. Agent selon la revendication 1, caractérisé en ce qu'il contient comme matière active l'éthoxycarbonylméthylester de l'acide 2- [4- (6-fluoroquinoxalinyl -2- oxy) -phénoxy] -propionique.

9. Agent selon la revendication 1, caractérisé en ce qu'il contient comme matière active le Méthoxycarbonylméthylester de l'acide 2- [4- (6-fluoroquinoxalinyl -2- oxy) -phénoxy] -thiopropionique.

10. Agent selon la revendication 1, caractérisé en ce qu'il contient comme matière active le 1-(méthoxycarbonyl) -éthylester de l'acide 2- [4-(6-fluoroquinoxalinyl -2- oxy) -phénoxy] -propionique.

11. Agent selon la revendication 1, caractérisé en ce qu'il contient comme matière active le n-butyloxycarbonylméthylester de l'acide 2- [4- (6-fluoroquinoxalinyl -2- oxy) -phénoxy] -thiopropionique.

12. Procédé de préparation des composés de formule I selon la revendication 1, caractérisé en ce que

a) on fait réagir un halogénure d'acide de formule II

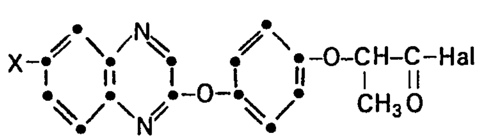

(II)

où X a la signification donnée pour la formule I et Hal représente un chlore ou un brome, éventuellement dans un solvant inerte et éventuellement en présence d'une base avec un ester d'acide alcanecarboxylique de formule III

H-Z-A-COOR (III)

où A, R et Z ont la signification donnée pour la formule I, ou

b) on fait réagir un chlorure de quinoxalinyle de formule IV

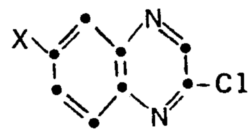

(IV)

où X a la signification donnée dans la formule I, dans un solvant inerte, éventuellement en présence d'une base, avec un ester d'acire phénoxypropionique de formule V

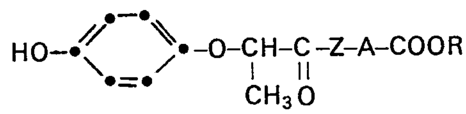

(V)

où A, R et Z ont la signification donnée pour la formule I, ou

c) on fait réagir un quinoxalinyl -2- oxyphénol de formule VI

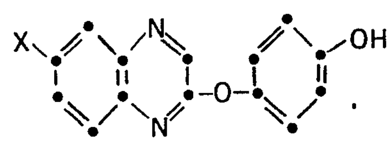

(VI)

où X a la signification donnée dans la formule I, dans un solvant inerte, éventuellement en présence d'une base, avec un ester d'acide propionique de formule VII

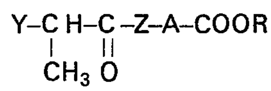

(VII)

où A, R et Z ont la signification donnée pour la formule I et Y représente un groupe sortant.

13. Procédé pour combattre la croissance végétale indésirable, caractérisé en ce qu'on fait agir une matière active de formule I selon la revendication 1, en une quantité efficace sur les plantes indésirables ou leur biotope.

14. Procédé selon la revendication 13 de lutte sélective contre les mauvaises herbes monocotylédones dans les plantes cultivées.

15. Application selon l'une des revendications 13 et 14, caractérisée en ce qu'on applique la matière active en post-levée.

16. Procédé pour influencer la croissance des plantes, caractérisé en ce qu'on fait agir une matière active de formule I selon la revendication 1 en une quantité efficace sur les plantes à influencer ou leur biotope.

17. Procédé selon la revendication 16 pour stimuler la croissance des racines dans les céréales.

18. Procédé selon l'une des revendications 16 et 17, caractérisé en ce qu'il s'effectue en pré-levée.

19. Procédé selon la revendication 17, caractérisé en ce qu'on traite les semences avec les matières actives avant l'ensemencement.

**Claims for the Contracting States: CH, DE, FR, GB, IT, LI**

1. A 2- [4- (6-Haloquinoxalinyl -2- oxy)-phenoxy]- propionic acid ester of the general formula I

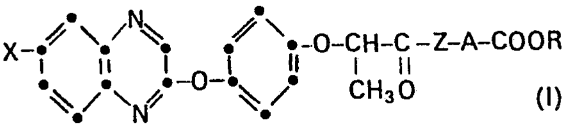

(I)

wherein X is fluorine, chlorine or bromine, Z is oxygen or sulfur, A is a $C_1$-$C_4$ alkylene bridge which is unsubstituted or substituted by methyl or ethyl, and R is $C_1$-$C_4$ alkyl, with the proviso that the –Z–A–COOR group is not $-O-CH(CH_3)-COOC_2H_5$.

2. A compound of the formula I according to claim 1, wherein X is fluorine or chlorine.

3. A compound of the formule I according to claim 1, wherein A is a $C_1$-$C_2$alkylene bridge which is unsubstituted or substituted by methyl or ethyl and which contains altogether not more than 3 carbon atoms.

4. A compound of the formula I according to either of claims 2 or 3, wherein X is fluorine or chlorine and A is a $C_1$-$C_2$alkylene bridge which is unsubstituted or substituted by methyl or ethyl and which contains altogether not more than 3 carbon atoms.

5. Methoxycarbonylmethyl 2- [4- (6-chloroquinoxalinyl -2- oxy) phenoxy] thiopropionate according to claim 1.

6. 1- (Ethoxycarbonyl) -n- propyl 2- [4- (6-chloroquinoxalinyl -2- oxy) -phenoxy] -propionate according to claim 1.

7. 1- (n-Butoxycarbonyl) -ethyl 2- [4- (6-chloroquinoxalinyl -2- oxy) -phenoxy] -propionate according to claim 1.

8. Ethoxycarbonylmethyl 2- [4- (6-fluoroquinoxalinyl -2- oxy) -phenoxy] -propionate according to claim 1.

9. Methoxycarbonylmethyl 2- [4- (6-fluoroquinoxalinyl -2- oxy) -phenoxy] -thiopropionate according to claim 1.

10. 1- (Methoxycarbonyl) -ethyl 2- (4- (6-fluoroquinoxalinyl -2- oxy) -phenoxy] -propionate according'to claim 1.

11. n-Butoxycarbonylmethyl 2- [4- (6-fluoroquinoxalinyl -2- oxy) -phenoxy] -thiopropionate according to claim 1.

12. A process for the preparation of a compound of the formula I according to claim 1, which process comprises

a) reacting an acid halide of the formula II

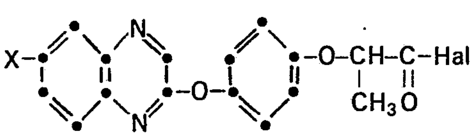

(II)

wherein X is as defined for formula I and Hal is chlorine or bromine, optionally in an inert solvent and optionally in the presence of a base, with an alkanecarboxylic acid ester of the formula III

H–Z–A–COOR (III)

wherein A, R and Z are as defined for formula, I, or

b) reacting a quinoxalinyl chloride of the formula IV

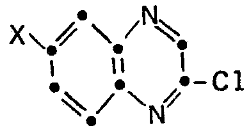

(IV)

wherein X is as defined for formula I, in an inert solvent and optionally in the presence of a base, with a phenoxypropionic acid ester of the formula V

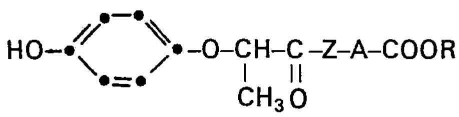

(V)

wherein A, R and Z are as defined for formula I, or

c) reacting a quinoxalinyl -2- oxy phenol of the formula VI

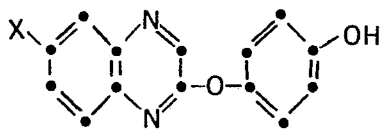

(VI)

wherein X is as defined for formula I, in an inert solvent and optionally in the presence of a base, with a propionic acid ester of the formula VII

Y–C H–C –Z–A–COOR (VII)
 | ||
 $CH_3$ O

wherein A, R and Z are as defined for formula I and Y is a leaving group.

13. A herbicidal and plant growth regulating composition which contains at least one compound according to claim 1, in addition to carriers and/or other adjuvants.

14. A method of controlling unwanted plant growth which comprises treating the unwanted plants or the locus thereof with an effective amount of a compound of the formula I according to claim 1.

15. A method according to claim 14, which comprises selectively controlling monocot weeds in crops of cultivated plants.

16. A method according to either of claims 14 or 15, wherein the compound is applied post-emergence.

17. A method of influencing plant growth, which comprises treating the plants to be influenced or the locus thereof with an effective amount of a compound of the formula I according to claim 1.

18. A method according to claim 17, which comprises stimulating the root growth of cereals.

19. A method according to either of claims 17 or 18 which is carried out preemergence.

20. A method according to claim 18, which comprises treating seeds with active ingredient before sowing.

**Claims for the Contracting States: BE, NL**

1. A 2- [4- (6-Haloquinoxalinyl -2- oxy) -phenoxy] -propionic acid ester of the general formula I

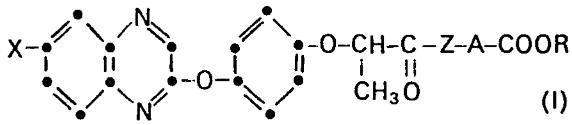

(I)

wherein X is fluorine, chlorine or bromine, Z is oxygen or sulfur, A is a $C_1$-$C_4$alkylene bridge which is unsubstituted or substituted by methyl or ethyl, and R is $C_1$-$C_4$alkyl.

2. A compound of the formula I according to claim 1, wherein X is fluorine or chlorine.

3. A compound of the formula I according to claim 1, wherein A is a $C_1$-$C_2$alkylene bridge which is unsubstituted or substituted by methyl or ethyl and which contains altogether not more than 3 carbon atoms.

4. A compound of the formula I according to either of claims 2 or 3, wherein X is fluorine or chlorine and A is a $C_1$-$C_2$alkylene bridge which is unsubstituted or substituted by methyl or ethyl and which contains altogether not more than 3 carbon atoms.

5. Methoxycarbonylmethyl 2- [4- (6-chloroquinoxalinyl -2- oxy) -phenoxy] -thiopropionate according to claim 1.

6. 1- (Ethoxycarbonyl) -n- propyl 2- [4- (6-chloroquinoxalinyl -2- oxy) -phenoxy] -propionate according to claim 1.

7. 1- (n-Butoxycarbonyl) -ethyl 2- [4- (6-chloroquinoxalinyl -2- oxy) -phenoxy] -propionate according to claim 1.

8. Ethoxycarbonylmethyl 2- [4- (6-fluoroquinoxalinyl -2- oxy) -phenoxy] -propionate according to claim 1.

9. Methoxycarbonylmethyl 2- [4- (6-fluoroquinoxalinyl -2- oxy) -phenoxy] -thiopropionate according to claim 1.

10. 1- (Methoxycarbonyl) -ethyl 2- [4- (6-fluoroquinoxalinyl -2- oxy) -phenoxy] -propionate according to claim 1.

11. n-Butoxycarbonylmethyl 2- [4- (6-fluoroquinoxalinyl -2- oxy) -phenoxy] -thiopropionate according to claim 1.

12. A process for the preparation of a compound of the formula I according to claim 1, which process comprises

a) reacting an acid halide of the formula II

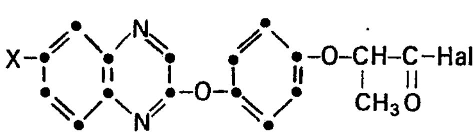

(II)

wherein X is as defined for formula I and Hal is chlorine or bromine, optionally in an inert solvent and optionally in the presence of a base, with an alkanecarboxylic acid ester of the formula III

H-Z-A-COOR (III)

wherein A, R and Z are as defined for formula I, or

b) reacting a quinoxalinyl chloride of the formula IV

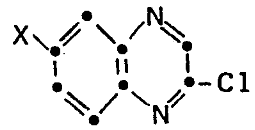

(IV)

wherein X is as defined for formula I, in an inert solvent and optionally in the presence of a base, with a phenoxypropionic acid ester of the formula V

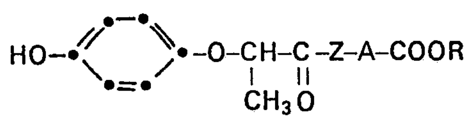

(V)

wherein A, R and Z are as defined for formula I, or

c) reacting a quinoxalinyl -2- oxy phenol of the formula VI

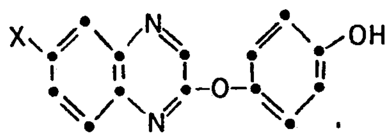

(VI)

wherein X is as defined for formula I, in an inert solvent and optionally in the presence of a base, with a propionic acid ester of the formula VII

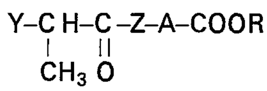

(VII)

wherein A, R and Z are as defined for formula I and Y is a leaving group.

13. A herbicidal and plant growth regulating composition which contains at least one compound according to claim 1, in addition to carriers and/or other adjuvants.

14. A method of controlling unwanted plant growth which comprises treating the unwanted plants or the locus thereof with an effective amount of a compound of the formula I according to claim 1.

15. A method according to claim 14, which comprises selectively controlling monocot weeds in crops of cultivated plants.

16. A method according to either of claims 14 or 15, wherein the compound is applied post-emergence.

17. A method of influencing plant growth, which comprises treating the plants to be influenced or the locus thereof with an effective amount of a compound of the formula I according to claim 1.

18. A method according to claim 17, which comprises stimulating the root growth of cereals.

19. A method according to either of claims 17 or 18 which is carried out preemergence.

20. A method according to claim 18, which comprises treating seeds with active ingredient before sowing.

**Claims for the Contracting State: AT**

1. A herbicidal and plant growth regulating composition which contains at least one 2- [4- (6-haloquinoxalinyl -2- oxy) -phenoxy] -propionic acid ester of the general formula I

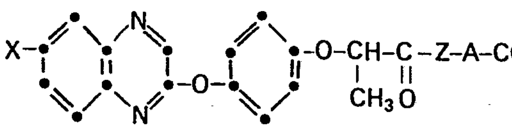
(I)

wherein X is fluorine, chlorine or bromine, Z is oxygen or sulfur, A is a $C_1$-$C_4$alkylene bridge which is unsubstituted or substituted by methyl or ethyl, and R is $C_1$-$C_4$alkyl, together with carriers and/or other adjuvants.

2. A composition according to claim 1, wherein X in the compound of formula I is fluorine or chlorine.

3. A composition according to claim 1, wherein A in the compound of formula I is a $C_1$-$C_2$alkylene bridge which is unsubstituted or substituted by methyl or ethyl and which contains altogether not more than 3 carbon atoms.

4. A composition according to either of claims 2 or 3, wherein X is fluorine or chlorine and A is a $C_1$-$C_2$alkylene bridge which is unsubstituted or substituted by methyl or ethyl and which contains altogether not more than 3 carbon atoms.

5. A composition according to claim 1, which contains methoxycarbonylmethyl 2- [4- (6-chloroquinoxalinyl -2- oxy) -phenoxy] -thiopropionate.

6. A composition according to claim 1, which contains 1- (ethoxycarbonyl) -n- propyl 2- [4- (6-chloroquinoxalinyl -2- oxy) -phenoxy] -propionate.

7. A composition according to claim 1, which contains 1- (n-butoxycarbonyl) -ethyl 2- [4- (6-chloroquinoxalinyl -2- oxy) -phenoxy] -propionate.

8. A composition according to claim 1, which contains ethoxycarbonylmethyl 2- [4- (6-fluoroquinoxalinyl -2- oxy) -phenoxy] -propionate.

9. A composition according to claim 1, which contains methoxycarbonylmethyl 2- [4- (6-fluoroquinoxalinyl -2-' oxy) -phenoxy] -thiopropionate.

10. A composition according to claim 1, which contains 1- (methoxycarbonyl) -ethyl 2- [4- (6-fluoroquinoxalinyl -2- oxy) -phenoxy] -propionate.

11. A composition according to claim 1, which contains n-butoxycarbonylmethyl 2- [4- (6-fluoroquinoxalinyl -2- oxy) -phenoxy] -thiopropionate.

12. A process for the preparation of a compound of the formula I according to claim 1, which process comprises

a) reacting an acid halide of the formula II

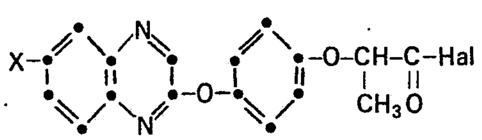
(II)

wherein X is as defined for formula I and Hal is chlorine or bromine, optionally in an inert solvent and optionally in the presence of a base, with an alkanecarboxylic acid ester of the formula III

H-Z-A-COOR (III)

wherein A, R and Z are as defined for formula I, or

b) reacting a quinoxalinyl chloride of the formula IV

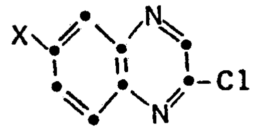
(IV)

wherein X is as defined for formula I, in an inert solvent and optionally in the presence of a base, with a phenoxypropionic acid ester of the formula V

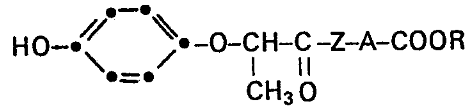
(V)

wherein A, R and Z are as defined for formula I, or

c) reacting a quinoxalinyl -2- oxy phenol of the formula VI

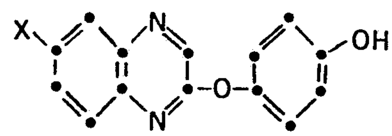
(VI)

wherein X is as defined for formula I, in an inert solvent and optionally in the presence of a base, with a propionic acid ester of the formula VII

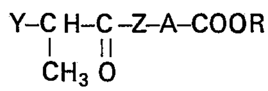
(VII)

wherein A, R and Z are as defined for formula I and Y is a leaving group.

13. A method of controlling unwanted plant growth which comprises treating the unwanted plants or the locus thereof with an effective amount of a composition according to claim 1.

14. A method according to claim 13 for the selective control of monocot weeds in crops of cultivated plants.

15. A method according to either of claims 13 or 14, wherein the composition is applied postemergence.

16. A method of influencing plant growth which comprises treating the plants to be influenced of the locus thereof with an effective amount of a composition according to claim 1.

17. A method according to claim 16, which comprises stimulating the root growth of cereals.

18. A method according to either of claims 16 or 17 which is carried out preemergence.

19. A method according to claim 17, which comprises treating seeds with the composition before sowing.